(19) Europäisches Patentamt
European Patent Office
Office européen des brevets

(11) Veröffentlichungsnummer: **0 220 412 B1**

(12) **EUROPÄISCHE PATENTSCHRIFT**

(45) Veröffentlichungstag der Patentschrift: **17.04.91**

(51) Int. Cl.5: **C07C 67/317**, C07C 69/74

(21) Anmeldenummer: **86111693.7**

(22) Anmeldetag: **23.08.86**

# (54) Verfahren zur Herstellung von reinen, chlorfreien Cyclopropancarbonsäureestern.

(30) Priorität: **26.10.85 DE 3538133**

(43) Veröffentlichungstag der Anmeldung:
**06.05.87 Patentblatt 87/19**

(45) Bekanntmachung des Hinweises auf die Patenterteilung:
**17.04.91 Patentblatt 91/16**

(84) Benannte Vertragsstaaten:
**AT BE CH DE FR GB IT LI NL SE**

(56) Entgegenhaltungen:
**GB-A- 2 008 110**
**GB-A- 2 061 925**
**US-A- 2 775 614**
**US-A- 4 590 292**

(73) Patentinhaber: **HÜLS AKTIENGESELLSCHAFT**
**- RSP Patente / PB 15 - Postfach 13 20**
**W-4370 Marl 1(DE)**

(72) Erfinder: **Kaufhold, Manfred, Dr.**
**Jasminweg 20**
**W-4370 Marl(DE)**

**EP 0 220 412 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von reinen, chlorfreien Cyclopropancarbonsäureestern der Formel 1 durch Umsetzung von Gamma-Butyrolacton mit Chlorwasserstoff und einem Butylalkohol zu einem Gamma-Chlorbuttersäureester 2 und anschließende Cyclisierung mit einem Alkoholat zu 1 sowie Umesterung von 1 in einen höher siedenden Ester 3,

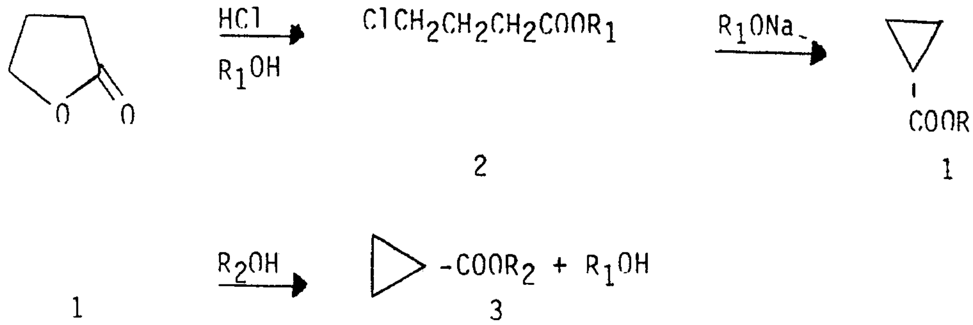


2 1

1 3

Synthesen von Gamma-Chlorbuttersäureestern und Cyclopropancarbonsäureestern, die vom leicht zugänglichen Gamma-Butyrolacton ausgehen, sind literaturbekannt.

So beschrieb bereits Reppe im Jahre 1955 die Synthese von Gamma-Chlorbuttersäuremethyl-, -ethyl-, -propyl- und -butylester als Eintopfververfahren durch Einleiten von Chlorwasserstoff in eine Lösung von Gamma-Butyrolacton und dem entsprechenden Alkohol und Zinkchlorid als Katalysator (Reppe, Annalen der Chemie 596 (1955), Seiten 163 bis 224). Infolge Bildung von Hochsiedern erzielte Reppe nur geringe Ausbeuten von z. B. 50,4 % im Fall des Methylesters.

Darum wurde in der DE-OS 27 51 134 ein Verfahren vorgeschlagen, das bei tiefen Temperaturen von 0 bis 20 °C und mit Thionylchlorid anstelle von Chlorwasserstoff arbeitet. Die Ausbeute an Methylester ist mit 91,3 % relativ hoch. Nachteilig bei diesem Verfahren ist jedoch neben dem hohen Preis des Thionylchlorids die Bildung von gasförmigem Schwefeldioxid während der Reaktion. Dieses Gas muß aus Umweltschutzgründen durch aufwendige technische Maßnahmen vernichtet werden.

Für die Cyclisierung von Gamma-Chlorbuttersäureester werden eine ganze Reihe von stark basischen Substanzen vorgeschlagen, wie z. B. Natriumamid (US-PS 3 294 833), Natriummethylat in Toluol (DE-AS 19 39 759 und DE-OS 27 51 133), Natrium-tert.-amylat (M u. S. Julia et al., Bull. Soc. Chim. Fr. 1960 , Nr. 2, Seiten 304 bis 312, Natriummethylat in Methanol (DE-PS 29 41 211) u. a.

In der zuletzt genannten Patentschrift, in der weitere Literaturstellen zitiert und diskutiert werden, wird festgestellt, daß man nur dann technisch interessante Ausbeuten erhalten kann, wenn man als Kondensationsmittel Natriumhydrid, Natriumamid oder alkoholfreie Alkoholate verwendet. Die Gegenwart von Alkoholen führt zu Nebenreaktion. Bei der Cyclisierung von Gamma-Chlorbuttersäureethylester mit Natriummethylat in Ethanol entsteht durch Substitution der 4-Ethoxi-buttersäureethylester als Nebenprodukt. Das analoge Problem soll beim Einstz der betreffenden Methylverbindungen auftreten.

Das Verfahren der DE-PS 29 41 211 löst das Ausbeuteproblem für den Einsatz der Methyl- und Ethylester durch Anwendung von hohen Temperaturen - beansprucht wird ein Temperaturbereich von 90 bis 200 °C - und Anwendung von Druck.

Dieser Temperaturbereich führt in dem System Gamma-Chlorbuttersäureethylester, Natriumamylat in t-Amylalkohol wie Julia et al. (s. o.) gezeigt haben nur zu einer geringen Ausbeute von 45 % d. Th. Aus den gesamten Literaturangaben ergibt sich somit die Lehre, daß die Gegenwart von Alkoholen bei der Cyclisierung stört - mit Ausnahme bei Anwendung der Systeme Natriumethylat in Ethanol und Natriummethylat in Methanol bei Temperaturen von 90 bis 200 °C.

Nachteilig bei diesen beiden Systemen ist aber, daß aufwendige Druckapparaturen benötigt werden und daß nach der Umsetzung und Zugabe von Wasser eine wäßrige Kochsalz, Alkohol und Reaktionsprodukte enthaltende Phase anfällt, die mittels mehrmaliger Extraktion aufgearbeitet werden muß.

Ein gravierender Nachteil bei dem Verfahren der DE-PS 29 41 211 ist zudem die zu geringe Reinheit der erhaltenden Cyclopropancarbonsäureester. Denn der Chlorgehalt dieser Ester liegt im allgemeinen bei 100 bis 1 000 ppm oder höher. Wegen dieser hohen Chlorgehalte können diese Ester nicht katalytisch zum Cyclopropylcarbinol hydriert werden.

Bei den Reaktoren würde Korrosion auftreten und der Hydrierkontakt würde vergiftet. Vielmehr muß der Chlorgehalt der für katalytische Hydrierungen vorgesehenen Ester unter 10 ppm liegen.

Für die Reduktion der chlorhaltigen Cycloppropancarbonsäureester kommen also nur kostspielige Reagenzien wie z. B. Lithiumaluminiumhydrid in Frage.

Alle bekannten Verfahren benötigen teure Chemikalien, Druckapparaturen und führen zu Problemen mit der Abfallbeseitigung. Wünschenswert ist daher ein Verfahren, bei dem Gamma-Butyrolacton im Eintopfverfahren mittels Chlorwasserstoff und einem Alkohol in einen Gamma-Chlorbuttersäureester übergeführt wird und dieser drucklos cyclisiert wird. Der entstandene Cyclopropancarbonsäureester kann beispielsweise katalytisch zum Cyclopropylmethanol hydriert werden.

Es besteht ein großes Interesse an einem derartigen Verfahren, nach dem man bei geringem technischen Aufwand und ohne den Einsatz von teuren Reagenzien aus Gamma-Butyrolacton Cyclopropancarbonsäureester und aus diesem gegegebenenfalls Cyclopropylcarbinol herstellen kann, weil diese Produkte wichtige Rohstoffe für Pharma-Produkte sind.

Die sich hieraus ergebende Aufgabe wird erfindungsgemäß entsprechend den Angaben der Patentansprüche gelöst.

Überraschenderweise erhält man in der ersten Reaktionsstufe bei der Umsetzung von Gamma-Butyrolacton mit Chlorwasserstoff und Butanol in Abwesenheit eines Katalysators sehr gute Ausbeuten von über 95 % an Gamma-Chlorbuttersäurebutylester, wenn man bei höheren Temperaturen Butanole und Chlorwasserstoff zum Gamma-Butyrolacton gibt und dabei Leichtsieder wie Butylchloride, Butanol und Wasser abdestilliert. Geeignete Butanole sind n-Butanol, iso-Butanol und tert.-Butanol. Bevorzugt setzt man n-Butanol ein. Die Umsetzung erfolgt bei 120 bis 140 °C in Abwesenheit eines Katalysators.

Die Cyclisierung des Gamma-Chlorbuttersäurebutylesters in der zweiten Reaktionsstufe gelingt überraschend glatt und mit guten Ausbeuten von ca. 95 %, wenn man bei höheren Temperaturen den Gamma-Chlorbuttersäurebutylester zu einer Lösung eines Natriumalkoholates in dem entsprechenden Alkohol, der 4 oder mehr C-Atome hat, gibt. Dieser Befund steht im Gegensatz zu den obengenannten Literaturstellen, die besagen, daß Alkohole mit Ausnahme von Methanol und Ethanol stören. Bevorzugte Alkoholate bzw. Alkohole sind Butanole, insbesondere n-Butanol, und Alkohole mit 5 bis 20, vorzugsweise 5 bis 10, C-Atomen und die entsprechenden Alkoholate. Die Verwendung einer Natrium-n-butylatlösung in n-Butanol ist besonders kostengünstig, weil sie aus Natronlauge und n-Butanol hergestellt werden kann.

Ferner wurde überraschenderweise gefunden, daß der destillierte Cyclopropancarbonsäurealkylester einen niedrigen Chlorgehalt von 10 ppm und kleiner hat, so daß eine weitere Reinigung, beispielweise vor der katalytischen Hydrierung, nicht erforderlich ist.

Um nach der Hydrierung ohne großen technischen Aufwand das Cyclopropylcarbinol destillativ gewinnen zu können, wird der Butylester gegebenenfalls in einer dritten Stufe mit einem höhersiedenden Alkohol mit mehr als 4 C-Atomen, insbesondere mit 5 bis 10 C-Atomen, umgeestert. Durch die Umesterung wird der Chlorgehalt weiter gesenkt, d. h. auf diese Weise ist eine Möglichkeit zur Reinigung von chlorhaltigen Produkten aus Zwischenläufen oder Fehlchargen gegeben.

Ein weiterer Vorteil des erfindungsgemäßen Verfahrens besteht darin, daß man Cyclisierung und Umesterung in einer Stufe als Eintopfverfahren durchführen kann, indem man während der Cyclisierung den höhersiedenden Alkohol mit mehr als 4 C-Atomen zugibt und Butanol dabei abdestilliert. Gibt man den höhersiedenden Alkohol bereits während der Cyclisierung zu, so kann man die zweite und dritte Reaktionsstufe somit in einer Stufe durchführen. (s. Beispiel 2). Das entstandene Natriumchlorid läßt sich durch Wasserwäsche leicht entfernen und das anfallende Abwasser hat wegen der geringen Wasserlöslichkeit der eingesetzten höheren Alkohole einen sehr geringen Kohlenstoffgehalt und braucht nicht aufgearbeitet zu werden.

Die praktische Durchführung der einzelnen Stufen erfolgt beispielsweise in folgender Weise:

Bei der Herstellung des Gamma-Chlorbuttersäure-n-butylesters wird ohne Katalysator gearbeitet, und zwar wird Gamma-Butyrolacton und n-Butanol im Molverhältnis 1 : 0,5 bis 1 : 5, vorzugsweise 1 : 1,0 bis 1 : 2 vorgelegt und unter Rückfluß zum Sieden erhitzt. Dann wird so schnell Chlorwasserstoffgas eingeleitet, wie das Gas aufgenommen wird. Über eine Kolonne werden Leichtsieder wie Alkylchloride, n-Butanol und Wasser ständig abdestilliert und das abgehende n-Butanol wird durch frisches ersetzt, so daß das Molverhältnis Gamma-Butyrolacton zu n-Butanol weitgehend konstant bleibt. Die Reaktionstemperatur beträgt vorzugsweise 126 bis 136 °C.

Die Cyclisierung des Gamma-Chlorbuttersäure-n-butylesters erfolgt bei 100 bis 200 °C, vorzugsweise bei 120 bis 140 °C, indem man beispielsweise eine Lösung von Natrium-n-butylat in n-Butanol vorlegt, den Gamma-Chlorbuttersäure-n-butylester zugibt und das Gemisch am Rückfluß zum Sieden erhitzt. Hierbei geht die Siedetemperatur von hohen Temperaturen, die der Alkoholat-Konzentration proportional sind, auf tiefere Temperaturen herunter, z. B. von 138 auf 122 °C. Das Molverhältnis Alkoholat zum Gamma-Chlorbuttersäure-n-butylester beträgt 1 : 1 bis 1,5 : 1, vorzugsweise 1,1 : 1 bis 1,2 : 1.

Der Überschuß an Alkoholat dient als Katalysator bei der gegebenenfalls direkt anschließenden

Umesterung. Hierbei wird der höhersiedende Alkohol etwa so schnell zugegeben, wie n-Butanol abdestilliert. Die Temperaturen im Reaktor werden durch den Siedepunkt des zugesetzten Alkohols bestimmt und liegen in dem Bereich von 120 bis 200 °C.

Das Molverhältnis Cyclopropancarbonsäurebutylester zu höhersiedendem Alkohol liegt bei 1 : 1 bis 1 : 5, vorzugsweise 1 : 1,1 bis 1 : 3.

Den erhaltenen Cyclopropancarbonsäureester setzt man beispielsweise ein, um durch Hydrierung das Hydroxymethylcyclopropan (Cyclopropylmethanol) zu erhalten. Hydroxymethylcyclopropan ist ein wichtiges Zwischenprodukt zur Herstellung von Pharma-Produkten. Die Hydrierung führt man bevorzugt in der Sumpf- und Rieselphase in Gegenwart eines Zn-Chromit-Katalysators bei 200 bis 350 °C und 200 bis 320 bar Wasserstoffdruck durch.

Den folgenden Beispielen ist weiteres Zahlenmaterial zu entnehmen; sie sollen das erfindungsgemäße Verfahren näher erläutern.

## Beispiel 1

a) Gamma-Chlorbuttersäure-n-butylester (1. Stufe)

Man benutzt eine Glasapparatur, die aus einem Dreihalskolben mit Gaseinleitungsrohr, Rührer, Thermometer, Tropftrichter und 0,5 m lange mit Glasraschigringen gefüllte Glaskolonne mit Destillationsaufsatz besteht.

Man setzt ein:

444 g (6 Mol) n-Butanol
352 g (4 Mol) Gamma-Butyrolacton (97,7 %ig)

Das Gemisch wird am Rückfluß zum Sieden erhitzt und sofort Chlorwasserstoff - 16 l/h - eingeleitet. Nach einer 1/2 h wird mit der Abnahme von Leichtsiedern begonnen. Der Verlauf der Synthese geht aus der folgenden Tabelle hervor:

| Zeit nach Beginn (h) | Temperaturen Sumpf (°C) | Temperaturen Kopf (°C) | Verhältnis Rücklauf zur Abnahme | HCl-Menge (l/h) | Zulauf n-Butanol gesamt (ml) | anfallendes Destillat gesamt (ml) |
|---|---|---|---|---|---|---|
| Beginn | 132 | 98 | - | - | - | - |
| 0,5 | 132 | 98 | 5 : 1 | 16 | - | 20 |
| 1 | 132 | 102 | 5 : 1 | 16 | - | 45 |
| 1,5 | 132 | 94 | 10 : 1 | 16 | - | 65 |
| 2 bis 3 | 130 | 93 | 20 : 1 | 16 | - | 80 bis 120 |
| 3,5 bis 5,5 | 128 bis 126 | 102 | 20 : 1 | 16 | - | 135 bis 190 |
| 6 | 128 | 102 | 20 : 1 | 8 | - | 200 |
| 6,5 bis 7,5 | 128 bis 136 | 102 | 20 : 1 | 8 | 50 bis 90 | 215 bis 250 |
| 8 bis 12 | 136 | 102 | 20 : 1 | 8 | 110 bis 250 | 260 bis 325 |
| 12,5 bis 15 | 136 | 102 | 20 : 1 | 8 | 280 bis 420 | 330 bis 395 |
| 15,5 | 136 | 117 | 3 : 1 | - | - | 405 |
| 16 | 136 | 110 | 3 : 1 | - | - | 440 |

Die Zugabe an n-Butanol wird so geregelt, daß die Sumpftemperatur nicht absinkt.

Gaschromatographische Analysen von Proben aus dem Sumpf nach 12 bis 16 Stunden zeigen folgende Gehalte an bekannten Verbindungen:

| Substanz: | Probe nach 12 h | Probe nach 16 h |
|---|---|---|
| n-Butylchlord | 0,3 % | 0,5 % |
| n-Butanol | 23,2 % | 24,8 % |
| Gamma-Butyrolacton | 0,8 % | 0,1 % |
| Chlorester | 72,0 % | 72,0 % |

Der Umsatz an Gamma-Butyrolacton liegt also nach 16 h bei 99,9 %.

Das bei der Umsetzung anfallende Destillat (387 g) mit einer Säurezahl von 275,8 wird mit 160 g 50 %iger Natronlauge neutralisiert und 100 g Wasser zur besseren Trennung zugegeben.

Abwassermenge: 386 g (C-Gehalt: 0,38 %)

Die Ölphase (248 g) hat nach GC-Analyse folgende Gehalte an bekannten Verbindungen:

| | | |
|---|---|---|
| n-Butylchlorid | | 14,7 % |
| n-Butanol | | 83,4 % |
| Lacton | kleiner | 0,1 % |
| Chlorester | kleiner | 0,1 % |

Als Sumpfprodukt fallen bei der Umsetzung 966 g an.

Aus dieser Zahl und den obengenannten Gehalten errechnet sich eine Ausbeute von Gamma-Chlorbuttersäure-n-butylester von 97,3 %, bezogen auf Einsatz.

Die Reindestillation liefert in einem Siedebereich von 93 bis 101 °C bei 13 mbar den Gamma-Chlorbuttersäure-n-butylester in 99,0 %iger Reinheit. Die Ausbeute an destilliertem Ester liegt bei 93 % d. Th., bezogen auf Einsatz.

Beispiel 1 b

b) Cyclopropancarbonsäure-n-butylester (2. Stufe)

Man benutzt eine Glasapparatur, die aus einem Dreihalskolben mit Rührer, Thermometer, Rückflußkühler und Tropftrichter besteht.

Man setzt ein:

718 g einer Lösung von Natrium-n-butylat in n-Butanol mit 2,2 Mol Na-n-butylat

Bei 138 °C wird innerhalb von 2 h unter Rückflußkochen

361 g (2,0 Mol) Gamma-Chlorbuttersäuren-n-butylester

(1. Stufe)

zugetropft. Hierbei geht die Temperatur gleichmäßig bis auf 122 °C herunter. Nach dem Eintropfen wird noch 2 h bei 122 °C gerührt. Nach dem Abkühlen wird mit Wasser gewaschen. Der Kohlenstoffgehalt des Abwassers liegt bei 0,9 %.

Die destillative Aufarbeitung liefert in einem Siedebereich von 111 bis 112 °C bei 133 mbar den Cyclopropancarbonsäure-n-butylester in 99,5 %iger Reinheit; sein Chlorgehalt liegt bei 10 ppm. Die Ausbeute an destilliertem Ester beträgt 268 g = 95 % d. Th., bezogen auf Einsatz.

Beispiel 1 c c) Umesterung (3. Stufe)

Man benutzt eine Glasapparatur, die aus einem Dreihalskolben mit Rührer, Thermometer und einer 0,5 m langen mit Glasraschigringen gefüllten Glaskolonne mit Destillationsaufsatz besteht.

Man setzt ein:

286 g (= 2,0 Mol) Cyclopropancarbonsäure-n-butylester

325 g (= 2,5 Mol) 2-Ethylhexanol

1,6 g (= 0,9 ml) konz. Schwefelsäure

Zunächst werden bei Normaldruck und Kopftemperaturen von 93 bis 117 °C und weiter bei 133 mbar und Kopftemperaturen von 66 bis 84 °C die gebildeten Leichtsieder, 152 g, über die Kolonne abdestilliert.

Nach dem Abkühlen werden zur Neutralisation der Schwefelsäure 2,8 g 50 %ige Natronlauge zum Sumpfprodukt gegeben und bei 13 mbar weiterdestilliert. Bei einem Siedepunkt von 114 °C (bei 13 mbar) fallen 360 g Cyclopropancarbonsäure-2-ethylhexylester mit 98 %iger Reinheit mit einem Chlorgehalt von nur 6 ppm an. Die Ausbeute ist demnach 91 % der Theorie, bezogen auf Einsatz.

Vergleichsbeispiel A

Die Durchführung erfolgt wie in der DE-PS 29 41 211 (Beispiel 1) beschrieben. Man benutzt einen 5 l-Rührautoklaven und setzt die in diesem Beispiel genannten Produkte in den angegebenen Mengen ein. Nach Aufarbeitung und Destillation erhält man 852 g Cyclopropancarbonsäuremethylester in einem Siedebereich von 114 bis 116 °C bei Normaldruck, d. h. die Ausbeute liegt bei 94,7 % und ist damit fast gleich wie in dieser DE-PS aufgeführt. Der Chlorgehalt des destillierten Cyclopropancarbonsäuremethylesters beträgt 8 000 ppm. Dieser Ester kann also nicht in die katalytische Hydrierung eingesetzt werden.

Beispiel 2

Nach dem Angaben des Beispiels 1 a) stellt man den Gamma-Chlorbuttersäure-n-butylester her.

Man benutzt die in Beispiel 1 c beschriebene Glasapparatur und setzt ein:
718 g einer Lösung von Natrium-2-ethylhexylat (2,2 Mol) in 2-Ethylhexanol
und erwärmt sie unter Rühren auf 130 °C. Dann werden innerhalb von 4 Stunden 360 g = 2 Mol Gamma-Chlorbuttersäure-n-butylester zugegeben. Nach der Zugabe wird die Temperatur auf 160 bis 164 °C erhöht, wobei sich Rückfluß einstellt und bei diesem Temperaturberich wird noch 2 Stunden gerührt.

Anschließend wird an der Kolonne Butanol abdestilliert und dabei die Sumpftemperatur bis auf ca. 200 °C erhöht.

Nach dem Abkühlen wird 2 mal mit Wasser gewaschen und zwar mit 400 bzw. 200 g Wasser. Der Kohlenstoffgehalt des Abwasser aus der 1. Wäsche (512 g) beträgt 1,5 % und der des Abwassers aus der 2. Wäsche (212 g) 1,2 %. Die destillative Aufarbeitung liefert in einem Siedebereich von 113 bis 114 °C bei 13 mbar den Cyclopropancarbonsäure-2-ethylhexylester in 99,1 %iger Reinheit mit einem Chlorgehalt von nur 6 ppm. Die Ausbeute an Destillat, 357 g entspricht einer Ausbeute von ca. 90 %, bezogen auf eingesetzten Gamma-Chlorbuttersäure-n-butylester.

## Ansprüche

1. Verfahren zur Herstellung von reinen, chlorfreien Cyclopropancarbonsäureestern durch Umsetzung von Gamma-Butyrolacton mit einem Alkohol und gasförmigem Chlorwasserstoff zum Gamma-Chlorbuttersäureester und dessen Cyclisierung mit einem Alkalialkoholat zum Cyclopropancarbonsäureester,
dadurch gekennzeichnet,
daß man zunächst in einer ersten Stufe Gamma-Butyrolacton und Butanol im Molverhältnis 1 : 0,5 bis 1 : 5 in Abwesenheit eines Katalysators bei einer Temperatur von 120 bis 140 °C mit Chlorwasserstoff umsetzt, wobei man über eine Kolonne Leichtsieder und Wasser abdestilliert und das abgehende Butanol durch frisches ersetzt, so daß das Molverhältnis Gamma-Butyrolacton zu Butanol weitgehend konstant bleibt,
anschließend in einer zweiten Stufe den in der ersten Stufe erhaltenen Gamma-Chlorbuttersäurebutylester mit einer Lösung eines Natriumalkoholats in dem entsprechenden Alkohol, der 4 oder mehr C-Atome hat, bei 100 bis 200 °C und einem Molverhältnis von Alkoholat zu Gamma-Chlorbuttersäurebutylester von 1 : 1 bis 1,5 : 1 cyclisiert,
und den in der zweiten Stufe erhaltenen Cyclopropancarbonsäureester gegebenenfalls in einer dritten Stufe mit einem höhersiedenden Alkohol, der mehr als 4 C-Atome hat, bei Temperaturen von 120 bis 200 °C umestert.

2. Verfahren nach Anspruch 1,
dadurch gekennzeichnet,
daß man in der ersten Stufe Gamma-Butyrolacton und Butanol im Molverhältnis 1 : 1 bis 1 : 2 einsetzt.

3. Verfahren nach Anspruch 1 und 2,
dadurch gekennzeichnet,
daß man Gamma-Butyrolacton mit Butanol und Chlorwasserstoff bei 126 bis 136 °C umsetzt.

4. Verfahren nach Anspruch 1,
dadurch gekennzeichnet,
daß man in der zweiten Stufe bei einem Molverhältnis Alkoholat zu Gamma-Chlorbuttersäure-butylester von 1,1 : 1 bis 1,2 : 1 cyclisiert.

5. Verfahren nach Anspruch 1 und 4,
dadurch gekennzeichnet,
daß man bei einer Temperatur von 120 bis 140 °C cyclisiert.

6. Verfahren nach Anspruch 1,
dadurch gekennzeichnet,
daß man zur Cyclisierung Gamma-Chlorbuttersäure-n-butylester und Na-n-butylat einsetzt.

7. Verfahren nach Anspruch 1,
dadurch gekennzeichnet,
daß man zur Cyclisierung Gamma-Chlorbuttersäure-n-butylester und das Natriumalkoholat eines höheren Alkohols mit mehr als 4 C-Atomen, vorzugsweise mit 5 bis 20 C-Atomen, insbesondere 5 bis 10 C-Atome, einsetzt und das anfallende n-Butanol abdestilliert.

8. Verfahren nach Anspruch 1,
dadurch gekennzeichnet,
daß man in der dritten Stufe mit einem Alkohol, der 5 bis 10 C-Atome hat, umestert.

## Claims

1. A process for the preparation of pure, chlorine-free cyclopropane carboxylic acid esters by the reaction of gamma butyrolactone with an alcohol and gaseous hydrogen chloride to give gamma chlorobutyric acid ester and its cyclization with an alkali alcoholate to give cyclopropane carboxylic acid ester,
characterized in that
in a first stage gamma butyrolactone and butanol in a molar ratio of 1 : 0.5 to 1 : 5 are reacted with hydrogen chloride in the absence of a catalyst at a temperature of 120 to 140 °C, low-boiling components and water being removed by distillation via a column and the butanol removed being replaced by fresh butanol, so that the gamma butyrolactone/butanol molar ratio remains substantially constant,
then in a second stage the gamma chlorobutyric acid butyl ester obtained in the first stage is cyclized with a solution of a sodium alcoholate in the corresponding alcohol, which has 4 or more carbon atoms, at 100 to 200 °C and with an alcoholate/ gamma chlorobutyric acid butyl ester molar ratio of 1 : 1 to 1.5 : 1,
and the cyclopropane carboxylic acid ester obtained in the second stage is if necessary re-esterified in a third stage with a higher-boiling alcohol which has more than 4 carbon atoms at temperatures of 120 to 200 °C.

2. A process according to claim 1, characterized in that in the first stage gamma butyrolactone and butanol are used in a molar ratio of 1 : 1 to 1 : 2.

3. A process according to claims 1 and 2, characterized in that gamma butyrolactone is reacted with butanol and hydrogen chloride at 126 to 136 °C.

4. A process according to claim 1, characterized in that in the second stage cyclization is performed with an alcoholate/gamma chlorobutyric acid butyl ester molar ratio of 1.1 : 1 to 1.2 : 1.

5. A process according to claims 1 and 4, characterized in that cyclization is performed at a temperature of 120 to 140 °C.

6. A process according to claim 1, characterized in that gamma chlorobutyric acid n-butyl ester and sodium n-butylate are used for the cyclization.

7. A process according to claim 1, characterized in that gamma chlorobutyric acid n-butyl ester and the sodium alcoholate of a higher alcohol having more than 4 carbon atoms, preferably 5 to 20 carbon atoms, more particularly 5 to 10 carbon atoms, are used for the cyclization, and the resulting n-butanol is removed by distillation.

8. A process according to claim 1, characterized in that in the third stage re-esterification is performed with an alcohol having 5 to 10 carbon atoms.

**Revendications**

1. Procédé de fabrication d'esters purs, débarrassés de chlore, d'acide cyclopropane carboxylique par transformation de gamma-butyrolactone avec un alcool et de l'acide chlorhydrique sous forme gazeuse, en un ester d'acide gamma chlorobutyrique et par cyclisation de celui-ci avec un alcoolat de métal alcalin, en un ester d'acide cyclopropane carboxylique, caractérisé en ce que d'abord, dans une première étape, on fait réagir la gamma -butyrolactone et le butanol dans un rapport molaire 1 : 0,5 à 1 : 5 en l'absence de catalyseur à une température de 120 à 140° C avec l'acide chlorhydrique, procédé dans lequel on sépare par distillation, dans une colonne, à bas point d'ébullition et l'eau, et que l'on remplace le butanol qui s'en va par du neuf, de façon que le rapport molaire gamma-butyrolactone/Butanol demeure largement constant, ensuite dans une deuxième étape ou cyclise l'ester butylique de l'acide gammachlorobutyrique obtenu au cours de la première étape avec une solution d'un alcoolat de sodium dans l'alcanol correspondant qui a 4 atomes de carbone ou plus, à une température allant de 100 à 200° C et pour un rapport molaire alcoolat/ester d'acide gammachlorobutyrique de 1 : 1 à 1,5 : 1 et que l'on transestérifie le cas échéant dans une troisième étape l'ester de l'acide cyclopropane carboxylique obtenu au cours de la deuxième étape, avec un alcool à point d'ébullition plus élevé, qui possède plus de 4 atomes de carbone, à des températures allant de 120 à 200° C.

2. Procédé selon la revendication 1, caractérisé en ce que l'on met en oeuvre dans la première étape la gamma-butyrolactone et le butanol dans un rapport molaire allant de 1 : 1 à 1 : 2.

3. Procédé selon les revendications 1 et 2, caractérisé an ce que l'on fait réagir la gammabutyrolactone avec le butanol et l'acide chlorhydrique à une température allant de 126 à 136° C.

4. Procédé selon la revendication 1, caractérisé en ce que l'on cyclise dans 1a deuxième étape avec un rapport molaire alcoolat/ester butylique d'acido de gamma-chlorobutyrique allant de 1 : 1 à 1,2 : 1.

5. Procédé selon la revendication 1 et la revendication 4, caractérisé en ce que l'on effectue la cyclisation à une température allant de 120 à 140° C.

6. Procédé selon la revendication 1, caractérisé en ce que l'on met en oeuvre pour la cyclisation de l'ester-n-butylique de l'acide gamma-chlorobutyrique et du n-butylate de sodium.

7. Procédé selon la revendication 1, caractérisé en ce que l'on met en oeuvre pour la cyclisation l'ester n-butylique de l'acide gamma-chlorobutyrique et l'alcoolat de sodium d'un alcool à poids moléculaire plus élevé ayant plus de 4 atomes de carbone, de préférence ayant de 5 à 20 atomes de carbone et en particulier de 5 à 10 atomes de carbone, et que l'on sépare par distillation le n-butanol qui est obtenu.

8. Procédé selon la revendication 1, caractérisé en ce que l'on transestérifie dans une troisième étape avec un alcool qui possède de 5 à 10 atomes de carbone.